# EUROPEAN PATENT SPECIFICATION

(11) **EP 1 858 427 B1**
(45) Date of publication and mention of the grant of the patent: **05.05.2010**
(21) Application number: 04797497.7
(22) Date of filing: 18.11.2004
(51) Int. Cl.: A61B 17/90

(54) **NEW FEMORAL TARGETING DEVICE FOR INTERNAL FIXATION OF FRACTURE FEMUR**
NEUE FEMORALE ZIELVORRICHTUNG FÜR DIE INNENFIXIERUNG EINER FEMURFRAKTUR
NOUVEAU DISPOSITIF DE CIBLAGE FEMORAL POUR LA FIXATION INTERNE D'UNE FRACTURE DU FEMUR

(43) Date of publication of application: 28.11.2007
(73) Proprietor: El Gamal, Mohamed Khairy, Nasr City, Cairo (EG)
(72) Inventor: El Gamal, Mohamed Khairy, Nasr City, Cairo (EG)
(74) Representative: Assendelft, Jacobus H.W.
(86) International application number: PCT/EG2004/000047
(87) International publication number: WO 2006/053573

(56) References cited:
- WO-A1-96/17556
- WO-A1-02/071963
- US-A- 2 500 993

## Description

The present invention relates to a Targeting Device for internal fixation of Fracture Femur. The method used for internal fixation of both Subtrochanteric and Supracondylar fracture femur, using condylar plate fixation. The method extends to be used for corrective Supracondylar Femoral Osteotomy.

Various methods and apparatus for Targeting Device for fracture fixation are known in the Orthopedic field. A method for condylar plate fixation is known from the field, using a femoral targeting device having a flap for temporary mounting to the shaft of the femor, a seating chisel guide and a hinge connecting both. This prior art has several drawbacks:
- The Flap:
   o The flap is short, so any minor changes in the angle will be much reflected upon the final result.
   o The flap is flat, so it does not accommodate the contour of the shaft of femur.
- The Hinge between the flap and the seating chisel Guide:
   o Although there is a way to adjust the angle, between the flap and the seating chisel, yet it is difficult to be precisely adjusted.
- The Seating Chisel Guide:
   o There is no way to precise introduce the guide wire in the middle of the seating chisel, and if so,
   o There is no guarantee to let the chisel go along the guide wire.
   o Finally, there is no way to be sure prior to chiseling that you are going to the right direction.

WO02071963 (document D1) and WO9617556 (document D2) also disclose a femoral targeting device, wherein however a hinge is absent such that the flap and seating chisel guide are rigidly connected. Different from WO02071963, WO9617556 discloses two guide wires on both sides of the chisel and each housed in a tube integral to the seating chisel guide: US2500993A (document D3) discloses the provision of a hinge to an internal fixation device for a fractured femur without the use of a femoral targeting device.

The new Femoral Targeting Device solves all the previous limitation of the conventional femoral targeting device by providing the features of claim 1. Further developments are provided by the subclaims.

Interlocking medullary nails have become the treatment of choice for nearly all femoral shaft fractures from the lesser trochanter to the femoral condyles. Most of pertrochanteric fracture femur is being treated by DHS. Other modalities of treatment include DCS and condylar plate fixation. Although condylar plate is a solid tension device yet it is relatively came out of reputation partly because of difficulty of the technique of fixation, especially in cases with fixing a long segment of bone. For this reason, efforts have been made for inventing a new targeting device for such Subtrochanteric / Supracondylar fracture femur, which called Femoral Targeting Device (FTD).

The New Targeting Device adapts the contour of the femur, and carries the advantage of precision and easy applicability. It consists of "Cannulated Guide Box" which is defined by boundary surfaces to be fitted inside the Seating Chisel Guide. Also it has "Long Contoured Flap" attached to the conventional upper part of the Seating Chisel Guide, by a hinge.

The "Cannulated Guide Box", has specific dimensions that fit inside the Seating Chisel Guide, which has two parallel cannulated guides along its whole length. The New Targeting Device can be easily assembled by being fitted to the back of the condylar plate during adjusting the correct angle between the Contoured Flap and the Seating Chisel Guide. This can easily be done by introducing the Seating Chisel along the Seating Chisel Guide to pass parallel to the blade part of the condylar plate, while the contoured Flap is snugly fitted to the plate part of the condylar plate.

### Brief Description of Drawings:

**Figure: 1** show assembled (Femoral Targeting Device) known as (FTD), which fits the contour of the femur (5). FTD consists of the proximal part of the conventional AO Seating Chisel Guide (2) (code# 332.09) "without its flap", the "Contoured Flap" (3), which fits the shaft of the femur and the "Cannulated Guide Box" (1), which fits inside the seating chisel guide (2), and accommodates two parallel guide wires (4). FTD should be fixed well with the shaft of the femur as by Bone Holding Forceps # (6).
**Figure: 2** show the Cannulated Guide Box (1) from different planes. It is cuboid in shape with specific dimensions (L x W x H) of (60 x 16 x 6) in mm, that fits inside the Seating Chisel Guide.
   Cannulated Guide Box contains two cannulated guide channels along its whole length (1A), with an inner diameter to accommodate for guide wires of 2mm in diameter (4). The distance between the two guide channels is about (10 mm), although they are parallel, yet they are not in the same level in the lateral plane i.e.; one is nearer to the upper surface and the other cannulated guide channel is nearer to the bottom surface, which has a clinical implications during operative procedure.
   Also the Cannulated Guide Box has two grooves along each side of the upper surface (1B), to be fitted well inside the Seating Chisel Guide of the AO System.
**Figure: 3** Show assembled Seating Chisel Guide (2) with the "Contoured Flap" (3), which is joined together by a hinge (3A). The upper part of the Contoured Flap is forming an angle with the rest of the shaft by 10 degrees. The angle between the Seating Chisel and the Contoured Flap can be adjusted by Hexagonal screw driver.
**Figure: 4** show combinations assembled FTD, with the Cannulated Guide Box in place within the Seating Chisel Guide and contain two guide wires (4). Different planes have been illustrated from the back side, bottom, top view and from the side view.
**Figure: 5** show the way of adjusting Femoral Targeting Device. This can be easily done by introducing the Seating Chisel (8) along the Seating Chisel Guide to pass parallel to the blade part of the condylar plate (7), while the contoured Flap is snugly fitted to the plate part of the condylar plate, so forming a mirror image to the condylar plate.

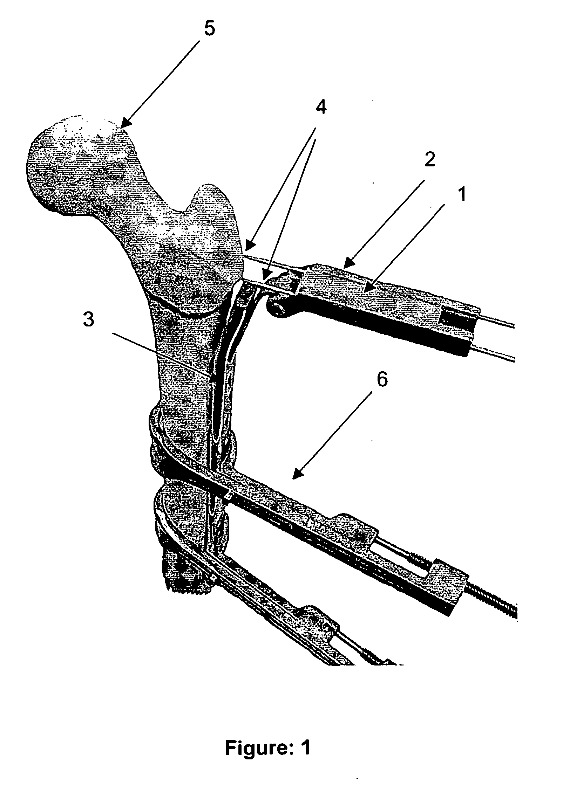

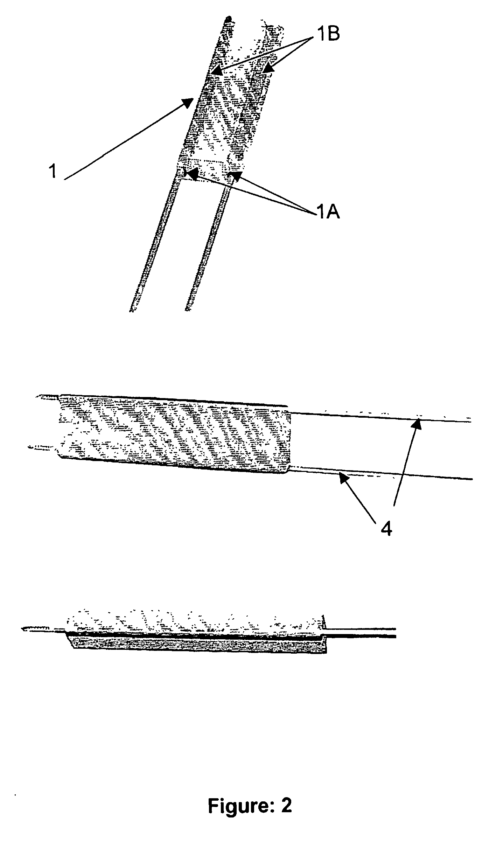

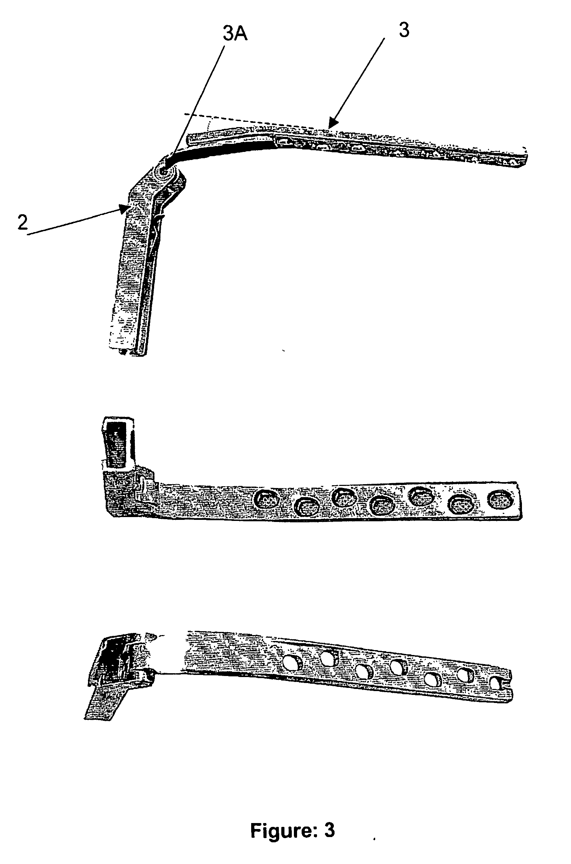

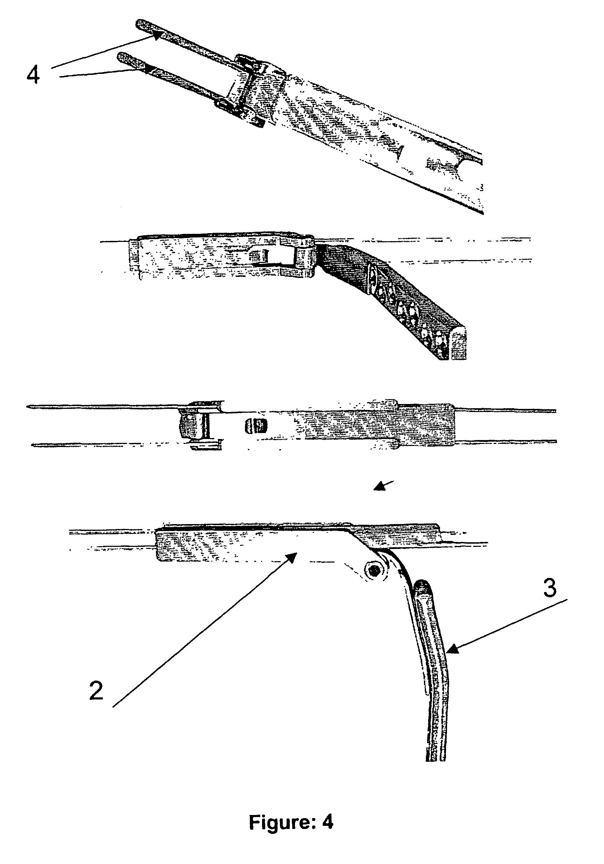

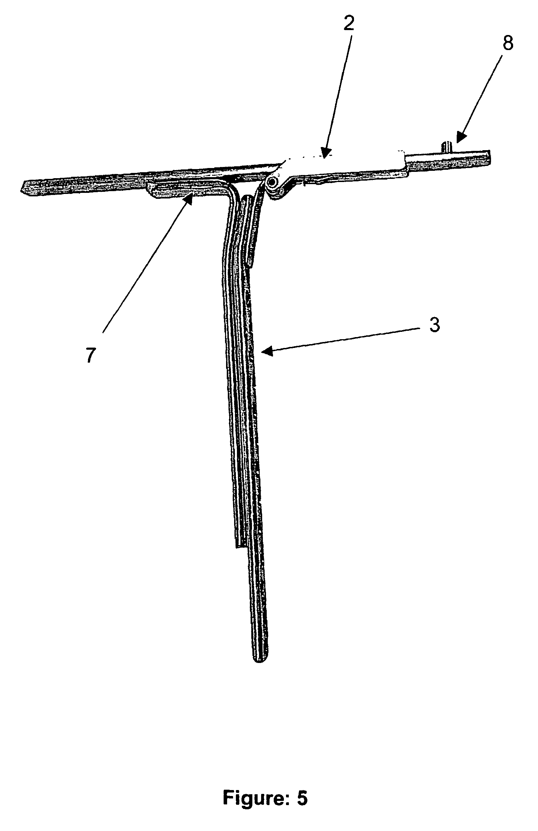

## Claims

1. A femoral targeting device which adapts to the contour of the femur, which consists of a Cannulated Guide Box (1) which is defined by boundary surfaces removably fitting inside a Seating Chisel Guide (2) and a Long Contoured Flap (3) is attached to the upper part of the Seating Chisel Guide (2) by a hinge (3A), said cannulated guide box (1) having two parallel cannulated guides (1A) along its whole length, with an inner diameter just to accommodate two guide wires (4).

2. Device according to claim 1, wherein the Cannulated Guide Box(1) is cuboid in shape and has specific dimensions, Length x Width x Height = 60 x 16 x 6 millimetres.

3. Device according to claim 1 or 2, wherein the two cannulated guides are not in the same level in the lateral plane, i.e. one is nearer to the upper surface and the other cannulated guide is nearer to the bottom surface.

4. Device according to claim 1, 2 or 3, wherein the inner diameter is just to accommodate a guide wire (4) of 2 millimetres in diameter.

5. Device according to claim 3 or 4, wherein the distance between the two cannulated guides is about 10 millimetres.

6. Device according to any of claims 2 - 5, wherein there are two grooves along each side of the upper surface of the cannulated guide box to fit well inside the Seating Chisel Guide.

7. Device according to any of claims 1 - 6 wherein the angle between the Seating Chisel Guide and the Long Contoured Flap shaft can be adjusted by using a hexagonal screw driver.

8. Device according to any of claims 1 - 7, wherein its upper part which is near to the hinge makes an angle with the rest of the shaft of the Long Contoured Flap by 10 degrees.

## Patentansprüche

1. Eine femoralen Zielgerät, die sich an die Kontur des Oberschenkels anpasst, die besteht aus einer kanülierten Führungsrahmen/Cannulated Guide Box (1), die von Grenzflächen definiert ist welche abnehmbar innerhalb ein Meißelführungsitz/Seating Chisel Guide (2) passen und ein lange konturierte Flansch/Long Contoured Flap (3) ist von ein Scharnier (3A) an den oberen Teil des Meißelführungsitz (2) befestigt, welche kanülierten Führungsrahmen (1) zwei parallelen kanülierten Führungen (1A) in seiner ganzen Länge hat, mit einem inneren Durchmesser um nur zwei Führungsdrahten (4) unterzubringen.

2. Vorrichtung nach Anspruch 1, wobei die kanülierten Führungrahmen (1) die Form eines Quaders hat und ganz bestimmte Abmessungen hat, Länge x Breite x Höhe = 60 x 16 x 6 Millimeter.

3. Vorrichtung nach Anspruch 1 oder 2, wobei die beiden kanülierten Führungen nicht auf der gleichen Niveau in der lateralen Ebene sind, d.h. die eine ist näher an der Oberfläche und die andere kanülierten Führung ist näher an der Unterseite.

4. Vorrichtung nach Anspruch 1, 2 oder 3, wobei der innere Durchmesser ausgelegt ist um nur einen Führungsdraht von 2 Millimeter Durchmesser auf zu nehmen.

5. Vorrichtung nach Anspruch 3 oder 4, wobei der Abstand zwischen den beiden kanulierten Führungen etwa 10 Millimeter ist.

6. Vorrichtung nach einem der Ansprüchen 2-5, wobei es zwei Nuten an jeder Seite der oberen Flache der kanülierten Führungsrahmen gibt, um eine gute Passform in dem Meißelführungsitz zu haben.

7. Vorrichtung nach einem der Ansprüchen 1-6, wobei der Winkel zwischen der Meißelführungsitz und der lange konturierte Flansch durch die Verwendung einer Sechskantschraubendreher angepasst werden kann.

8. Vorrichtung nach einem der Ansprüchen 1-7, wobei seinem oberen Teil, die das Scharnier in der Nähe ist, einen Winkel mit dem Rest der Welle der lange konturierte Flansch um 10 Grad hat.

## Revendications

1. Un dispositif de ciblage de fémorale qui s'adapte à la courbure du fémur, qui se compose d'un cadre de guide canulées/Cannulated Guide Box (1), qui est définie par les surfaces limites amovible fixe à l'intérieur d'un siège de guide de ciseau/Seating Chisel Guide (2) et une bride longue profilée/Long Contoured Flap (3) est relié à la partie supérieure du siège de guide de ciseau (2) par une charnière (3A), quelle cadre de guide canulées comportant deux guides (1A) canulées parallèles sur toute sa longueur, d'un diamètre intérieur, juste pour accueillir deux câbles de guide (4).

2. Dispositif selon la revendication 1, dans lequel la cadre de guide canulées (1) est cuboïde en forme et a des dimensions spécifiques, Longueur x Largeur x Hauteur = 60 x 16 x 6 millimètres.

3. Dispositif selon la revendication 1 ou 2, dans lequel les deux guides canule ne sont pas dans le même niveau dans le plan latéral, à savoir un est plus proche de la surface supérieure et l'autre guide canulées est plus près de la surface inférieure.

4. Dispositif selon la revendication 1, 2 ou 3, dans lequel le diamètre intérieur est juste de recevoir un fil de guide (4) de 2 millimètres de diamètre.

5. Dispositif selon la revendication 3 ou 4, dans lequel la distance entre les deux guides canulées est d'environ 10 millimètres.

6. Dispositif selon l'une des revendications 2-5, dans lequel il y a deux rainures de chaque côte de la surface supérieure de la cadre de guide canulées pour s'adapter à bien à l'intérieur du siège de guide de ciseau.

7. Dispositif selon l'une des revendications 1-6, dans lequel l'angle entre la siège de guide de ciseau et la bride longue profilée peut être ajusté à l'aide d'un tournevis hexagonal.

8. Dispositif selon l'une des revendications 1-7, dans lequel sa partie supérieure qui est près de la charnière fait un angle avec le reste de l'arbre de la bride longue profilée par 10 degrés.
